# EUROPEAN PATENT APPLICATION

(11) **EP 1 949 906 A1**
(43) Date of publication of application: **30.07.2008**
(21) Application number: 06799669.4
(22) Date of filing: 23.08.2006
(51) Int. Cl.: A61K 38/08, A61K 38/07, A61K 45/06, A61P 25/28

(54) **AGENT FOR CORRECTING STRESS-INDUCING NEURO-MEDIATOR, NEURO- ENDOCRINE AND METABOLIC DISTURBANCES AND METHOD FOR PREVENTING AND TREATING CONCOMITANT PATHOLOGICAL CONDITIONS**

(30) Priority: 08.09.2005 RU 2005128993
(71) Applicant: Obschestvo S Ogranichenoy Otvetstvennost Ju Issledovatelsky Tsentr "Komkon", St. Petersburg 197042 (RU)
(72) Inventor: MIKHALEVA, Inesa Ivanovna, Moscow, 117437 (RU); IVANOV, Vadim Tikhonovich, Moscow, 111296 (RU); PRUDCHENKO, Igor Arkadjevich, Moscow, 117378 (RU); VOITENKOV, Boris Olegovich, St.Petersburg, 195271 (RU)
(74) Representative: Henrion, Oliver
(86) International application number: PCT/RU2006/000468
(87) International publication number: WO 2007/030035

(57) **Abstract**

The invention relates to medicine, in particular to pharmacology, and is embodied in the form of an agent for limiting neuro-mediator, neuro-endocrine and metabolic disturbances generating the disorders of a central nervous system and functional somatic disorders. The inventive agent is based on a DSIP (delta sleep inducing peptide) or a derivative chemically related thereto and can be used for correcting and preventing functional shifts in a central nervous system and concomitant somatic disorders caused by unfavourable and extreme environmental factors and different pathological conditions of an organism or during the ageing thereof. The inventive method for preventing and treating stress states with the aid of the inventive agent is **characterised in that** the inventive compostions can be administrated: intranassally in the form of intranasal drops or spray; sublingually in the form of resorbable tablets and capsules; in the form of powders, suppositories, ointments and creams; in the form of different injections (intracutaneous, hypodermic, intramuscular and intravenous); in the form of different cosmetic agents (creams, lotions, tonics, cosmetic milk, foam, soap, etc.) and in the form of infant food components.

## Description

The invention refers to medical science, in particular to pharmacology. Specifically the present invention represents an agent that is capable of limiting neurotransmitter, neuroendocrinal and metabolic disorders leading to central nervous system disorders and functional somatic abnormalities. Said disorders evolve from various external stress influences as well as from different diseases, organic disorders and also during the process of aging. Moreover, the agent that is the subject of the present invention can provide prophylaxis and both prevent the onset of functional disorders and attenuate their extent, to affect positively the adaptation of an organism and its resistance to deleterious stress factors.

### List of the used abbreviations:

- CNS: the central nervous system
- DSIP: delta sleep inducing peptide
- FDSIP: family of peptides inducing delta sleep
- GABA: gamma-amino butyric acid
- NMDA: N-methyl-D-aspartate
- NAD: nicotine amide dinucleotide
- ECG: Electrocardiography
- TBI: traumatic brain injury
- ICP: infantile cerebral paralysis

The agent to be patented prophylactic against the development of psycho-emotional and systemic stress, is stabilizing CNS functions and is preventive against massive neuronal death under conditions of adaptation reaction of the organism to extreme stress influences (stress), including direct affects to the organism caused by deleterious factors of different nature (radial, chemical, infectious, traumatic, etc.) and indirect stress influences accompanying organism responses to the risk of damage or to life-threatening conditions (loss of water or food resources, birth, reproducibility). The agent can be used for the treatment and for the prevention of diseases in humans, including children, comprising neonates, and in animals.

During the recent years it has been clearly demonstrated that the adaptation reaction (stress) develops in response to threat or damage of the organism itself and/or to the threat of basic biologic requirements of the organism (saving of life, birth, reproducibility and access to energy resources). In the case of great intensity and/or long duration of stress, not only a functional, but also a morphological damage of the CNS develops, which manifests in an extensive loss of neurons (1, 2).

The result of stress-induced damage of the CNS is the development of an entire complex of pathological processes including:
■ disruption of activity of main systems of homeostasis maintenance being:

■ neuroendocrine system;
■ nervous system;
■ immune system;
■ metabolic control;
■ cardiovascular, respiratory and intestinal system, and also
■ massive damage to cells and tissues caused by free radicals (2).

In result of the abovementioned processes an entire complex of the so-called stress-associated-diseases develops in the stressed organism with a wide range of acute and chronic pathologies. For example, a pathogenic role of stress in ischemic heart and brain disease, ulcer lesions of the gastrointestinal tract, aggravation of allergic and infectious diseases, etc. was shown. Along with acute stress consequences also long-term effects occur beginning with the posttraumatic stress-syndrome (or posttraumatic stress reaction) to a significant reduction of life span and massive intensification of cancerogenesis. Consequences of the mutagenic effect of damage caused by free radicals to fetal organisms that are carried to term under conditions of stress, including systemic stress caused by a deficit of mother's nutrition have been shown (3, 4, 5).

Thus, the stress reaction causes damage to main systems of an organism and leads to the development of a number of pathologies, e.g. to the aggravation of existing diseases, to a reduced resistance of the organism and to genomic damage.

Such a deep and universal character of the damage caused by stress makes the search of effective agents for limiting the consequences of the adaptation syndrome a very acute problem.

Until recently, various representatives of benzodiazepine agents, antidepressants, antioxidants, nootrops, vitamin complexes and a number of other pharmacological agents were used as anti-stress agents (6).

However, the majority of these substances show either a superficial symptomatic effect or a wide range of unwanted side effects including a depressing effect on the CNS function. Moreover, psycho-emotional and systemic stresses are virtually constant or frequently repeating events and accordingly anti-stress agents should be used frequently or for a long time period resulting in the appearance of unwanted side effects caused by the known anti-stress agents, which lead to extremely negative consequences. Therefore, there is an urgent need for the development of effective anti-stress agents having a pathogenetic character of action, which are non-toxic, and do not depress the CNS function or cause addiction. Agents of endogenous origin playing a certain physiological role in the organism have the above-described set of properties. The most studied representative of said class of substances is a complex of endogenous peptide regulators including the family of DSIP peptide regulators.

It is known that endogenous bioregulator factors directly participate in the realization of the highly complex mechanisms of physiological regulation processes and homeostasis in human and animal organism. Endogenous regulatory peptides are the important components of the peptidergic regulation system and show polyfunctional and prolonged actions in the organism. One of these endogenous peptides is delta-sleep inducing peptide (DSIP) (or Trp-Ala-Gly-Gly-Asp-Ala-Ser-Gly-Glu, delta sleep inducing peptide) (7).

Various stress-protective and "normalizing" effects of the delta-sleep peptide are well studied in animals and have already been described in several tens of publications, where its uniqueness has been credibly shown and the mechanisms of its action have been studied (8). Results of studies with DSIP and its analogues obtained by us and by other authors allow to positively confirm that it is the representative of a family of peptide regulators, that stabilize the activity of the CNS under conditions of the adaptation syndrome (9).

The present invention refers to compounds of peptidic nature possessing pharmacological activity, which can be used for the treatment and the prophylaxis of a wide range of stress-induced pathologies either caused by the effects of various disturbing factors or originating in result of different diseases and traumata that lead to pathologic disorders of function of organs, tissues and systems of the organism. A disorder of neurotransmitter and neurohumoral balance, and thus of the balance of neuronal activation-inhibition processes under the influence of acute and chronic negative effects has an essential pathogenetic significance for the subsequent development of functional and organic somatic disorders and stress-induced diseases. The agent of the present invention is intended for the compensation of an deficit of endogenous peptide regulators of the DSIP family, the deficiency of which under conditions of severe and/or continuous stress leading to a toxic brain damage in the early postnatal period, in the aging process and in case of intoxications of different etiologies.

The present invention provides an agent based on DSIP or its derivatives chemically related to DSIP, which is suggested for the correction and prophylaxis of functional changes in the central nervous system and accompanying somatic disorders, which occur due to negative and extreme environmental factors as well as during different pathologic conditions and during aging.

The invention relates to pharmaceutical compositions based on the endogenous bioregulator peptide known as delta-sleep peptide (delta sleep inducing peptide DSIP), on its analogues and its derivatives comprising one or more of said compounds in combination with pharmaceutically acceptable additives and carriers. The agent represents a composition of agents of peptidic or other nature, comprising peptides of the DSIP family (FDSIP) (10, 11).

Diseases associated with the exposure to various stress factors like emotional, physical, psychological or pathophysiological factors represent a huge and heterogeneous group of acute and chronic pathological conditions of human or animals, which provide the basis for various central and peripheral functional disorders as a result of neurotransmitter, neuroendocrinal and metabolic abnormalities. The agent is used for the compensation of a deficiency of an endogenous peptide regulator of the DSIP family the presence of which in the organism provides the possibility of an adaptive modulation of the balance of inhibitory and excitatory neurotransmitter levels to the organism. Deficiency of endogenous DSIP develops under conditions of severe or continuous stress, in case of toxic brain damage, in particular alcoholic damage, in the early postnatal period, in the aging process, and also in the case of individual hypersensitivity to stress and reduction of adaptive capabilities. As a result of the administration of an endogenous peptide regulator of the DSIP family a cascade of integrative neurochemical alterations proceeds in the organism, providing an adaptive modulation of the balance of inhibitory and excitatory neurotransmitter level, of neurohormones and bioregulators in brain structures and in the periphery of nerve terminals.

Contrary to many classic drugs used for reduction of stress-induced pathologies the present agent is physiological, non-toxic and at the same time is effectively normalizing impaired organism functions, preventing or attenuating disorders of said functions.

The present agent can be used for curative and the prophylactic purposes in form of a monotherapy or together with other drugs as a regulator of different metabolic, neurohumoral and endocrinal disorders caused by external and internal disturbing factors, more specifically:
as stress-protector, adaptogene, neuroprotector, immunocorrector, geroprotector, antioxidant, antitoxicant, anticonvulsive, agent for the treatment and prophylaxis of alcoholism, narcotic and drug addiction, abstinence syndrome, sleep disorders, discirculatory encephalopathies, neurosis, vegetative disorders, memory and attention disorders, depressive conditions and cardiovascular pathologies, brain blood circulation disorders, autoimmune diseases and endocrinal disorders.

As is known, compounds of peptidic nature are usually not suitable for the application per os due to their ready enzymatic degradation inside the gastrointestinal tract. Intranasal or sublingual dosage forms of the present agents are particularly preferred as they provide rapid penetration of the drug into the brain through the nose mucosa or in case of the sublingual application into the blood, bypassing the gastro-intestinal tract and the liver. These methods of administration allow the usage of lower doses of active substances compared to an injective administration, besides the avoidance of other known disadvantages of injection administration (infection risk and painfulness, necessity of medical personnel participation, etc.). However, the injection administration of the present agents is also suitable as the active components are able to cross the blood-brain barrier and to penetrate from blood vessels into the brain.

The mechanisms of the physiological action of FDSIP peptides and the resulting pharmacological efficacy of the use of the present medical compositions are briefly discussed below and are demonstrated by a series of examples. Others and we demonstrated in various studies that DSIP under stress provides a modulatory action on the balance of activation and inhibition processes in the brain by changing the level of excitatory and inhibitory neurotransmitters such as GABA, glutamic and aspartic acid and serotonin. According to our data the modulatory effect on the CNS by FDSIP peptides is realized by the modulation of the activity of extremely important neuronal receptor complexes (12, 13, 14).

Particularly, the evaluation of the GABA-effecting component in the mechanism of action of the peptidic composition "Deltaran" based on FDSIP has been performed using the "pharmacological profiling" method in animals (mice). In result of experiments on specific convulsive and comatose models said peptidic drug shows pronounced affinity to the GABA_{A}-receptor complex. It was found that FDSIP peptides modulate the activity of the GABA-benzodiazepine-ionophore-receptor complex by increasing the sensitivity to the respective ligands and by potentiation of their action, thus providing regulatory influence of inhibitory processes in the neuron. Data have been obtained that show a significant modulatory influence of the peptide drug on the benzodiazepine fragment of the GABA-benzodiazepine-ionophore-receptor complex against the background of norbornan-pyrotoxin kindling likely as a result of an increase of agonist affinity to the binding site and enhancement of its effect on the entire organism. Also evidence has been found for a modulation of the barbiturate binding site of the GABA-benzodiazepine-ionophore-receptor complex by Deltaran and DSIP and data have been obtained concerning regulatory effect on chloride ionophore. The regulatory influences are probably realized via the peptide fragment of the GABA-benzodiazepine-ionophore-receptor complex (15).

The neurotropic effects of peptides of the DSIP family to a considerable extent are due to their capability to limit an excessive neuronal excitability, hyperexcitability, caused by the overproduction of the excitatory neurotransmitter glutamate during stress exposure and traumas. FDSIP peptides modulate the GABA-receptor activity by increasing the sensitivity to the respective ligand and thereby enhancing the inhibitory processes in the neuron. The hyperactivation of glutamate receptors of the NMDA-subtype causes a cascade of biochemical events leading in result to neuronal death. Likewise, FDSIP having glutamate at the C-terminal additionally act on the NMDA-receptor limiting its responsiveness to excitatory signals of glutamate. Such complex influence significantly limits hyperexcitation induced by glutamate and other excitatory neurons effects and mediators, and also limits application of excitatoxic mechanisms, whereas oxidative stress being an inducer of apoptosis is of essential importance in realization of said mechanisms. The pathogenesis of severe cardiovascular squeals, hyperglycemia, epilepsy, premature aging, neurodegenerative and oncologic diseases is based on the occurring condition of acute or chronic excitotoxicity (10).

An important part of the stress-protective mechanism of the peptides of the DSIP family is their indirect and very effective antioxidant action, specifically under stress conditions. The reduction of stress induced overaccumulation of active forms of oxygen leads to a restriction of multiple destructive sequelae of chronic or acute stress. It is known that in different cell types the intracellular oxidative - reductive balance is tightly controlled. An impairment of this balance results in serious consequences for the cells due to the modulation of gene expression and following metabolic changes. According to the common knowledge a significant defect in pro- and antioxidant balance results in cell death by apoptosis or necrosis due to a damage of virtually all important macromolecules, including nucleic acids, proteins, carbohydrates and lipids caused by active forms of oxygen and nitrogen(16).

A number of biochemical and biophysical experiments have clearly shown an inhibitory and preventive action of delta-sleep inducing peptide, deltaran and other FDSIP peptides on the peroxidation of lipids of various biological membranes (synaptosome, mitochondrial, erythrocyte and other) by modulation of the activity of antioxidant enzymes. The direct effect of the peptide on different cell membrane types was shown which may provide an additional normalizing cell functions effect. Particularly FDSIP may provide a stabilizing effect to the mitochondrial cell apparatus that also is capable of reducing cell damage by normalizing the cell respiration processes, which has been shown by us in experiments. Also a significant reduction of the stress-induced accumulation of free radicals in different tissues of the organism was found. So administration of exogenous DSIP credibly increases the capacity of the antioxidant systems of the liver, the brain and the blood under normal and under stress conditions, and increases the capacity of the endogenous enzymatic (glutathione-dependent enzymes, superoxide dismutase, catalase) and non-enzymatic (low molecular weight antioxidants, such as urea, uric acid) antioxidant protection systems (17, 18).

DSIP and its related peptides play a unique role in the maintenance of the structural-metabolic homeostasis not only on the level of different organs and tissues but also on the sub-cellular level of the organism. The specific interaction of exogenous DSIP with plasma membranes of human blood cells (erythrocytes and thrombocytes) and synaptosomal membranes (mice brain) causes an increased mobility of structural membrane domains on the surface and on its internal non-polar region. Such a decrease of rigidity of biological membranes enables the normalization of membrane functions, that have been disturbed by manifestations of the metabolic syndrome, such as hypertonic disease, hyperinsulinemia, misbalance of the endocrinal system, aging, etc. In studies of the influence of DSIP and Deltaran on the activity of the mitochondrial respiratory chain and on the efficacy of the oxidative phosphorylation performed by us and others on rat brain preparations a significant increase in the efficacy of the oxidative phosphorylation has been shown. Using the experimental hypoxia model it was shown that a preliminary administration of small doses of deltaran completely inhibited a decrease in the efficacy of oxidative phosphorylation caused by experimental hypoxia (15).

DSIP is able to modulate the activity of a number of membrane-associated key enzymes including mitochondrial enzymes controlling the metabolism in brain and peripheral tissues: monoamine oxidase A, hexokinase, NAD-dependent malate dehydrogenase, creatine kinase, all enzymes of the respiratory chain, hypothalamic glutamine synthetase, superoxide dismutase of the rat liver and membrane enzymes of the adenosine metabolism in rat peritoneal macrophages (19, 9).

A large number of studies confirmed the stress-protective and adaptogenic action of delta-sleep peptide is independent of the stress type or other pathological influences. Delta-sleep peptide shows properties of the endogenous factor by reducing or even preventing, , stress-induced pathological disorders, which promotes retention of the biological and physiological processes within the scope of adaptive norm. This unusual membrane-active neuropeptide is a natural adaptogen. The characteristic feature of DSIP action is its capability of modulating central regulatory processes. DSIP limits the pathological disturbance of said central regulatory processes during the exposure to various external and internal disturbing factors. Clinically extra relevant is its ability to show maximal efficacy during stress-induced disturbances and to cause no or only minor effects in the absence of stress-inducing factors (20).

An agent for reducing side effects associated with the complex and combined treatment of malignant neoplasia, the treatment of virus hepatitis and also the side effects induced by antitumor and other chemical agents with simultaneous attenuation of paraneoplastic syndrome, comprising the nonapeptide DSIP having an amino acid sequence Trp-Ala-Gly-Gly-Asp-Ala-Ser-Gly-Glu, the dipeptide carnosine and glycine amino acid at weight ratios of the components of 1: (1-100): (1-100), is known (21). However said agent and the method of prophylaxis and treatment using this agent are insufficiently universal.

Another agent is known for modulating the N-methyl-D-aspartate (NMDA) receptor response by means of S-substituted glutathione derivatives. This agent is intended for the reduction of pain and treatment of neurological diseases and traumas of the nervous system in mammals, using components affecting and modifying one of the oxidative sites of the N-methyl-D-aspartate (NMDA) receptor in mammals. These components include S-substituted glutathione derivatives that modulate and regulate the NMDA glutamate receptor. A method of treatment of diseases and pathological conditions of the central nervous system in mammals comprising administration of therapeutically effective amounts of a substance that interacts with and modifies one of the oxidative sites of NMDA receptor in mammals one of the components of said substance being S-substituted derivatives of glutathione - is known from (22). The above described agent is not universal neither by the mechanism of action, nor by the scope of use.

A method of treatment of ischemic insults by melatonin administration is also known. The development of acute neurological symptoms being a possible manifestation of insult is also considered to be acute cerebrovascular pathology. The method of treatment consists in the administration of therapeutically effective amounts of melatonin immediately after the development of acute neurological symptoms in case of a neurological defect. The optimal time interval for drug administration is within the first three hours of the occurrence of the neurological defect. The therapeutically effective amount of melatonin is ranging from not less than 200 mg to not more than 1000 mg. For newborn children and children under five years of age the effective amount of melatonin is less than 200 mg and for adults with a high body weight the melatonin dose can exceed 1000 mg. Generally, the effective melatonin amount does not exceed 1500 mg in most cases. The need of administration of therapeutically effective doses of melatonin immediately after development of neurological symptoms is a disadvantage of this method, wherein the effective dose of melatonin is not less than 200 mg and does not exceed 1500 mg (23).

The closest prior art with respect to the present invention is an invention of a Russian patent being a drug showing antistress, stress-protective and nootropic action, which is also used for the treatment of alcoholism and drug addiction. Said drug is characterized in that it contains synergistically acting ingredients: nonapeptide DSIP with the amino acid sequence Trp-Ala-Gly-Gly-Asp-Ala-Ser-Gly-Glu and glycine at a weight ratio of the components of 1:1 to 1:100. A method of prophylaxis and treatment of stress conditions by administering a glycine containing drug is characterized in that the drug is administered intranasally in a dose of 3 - 9 mg / day for 1 - 3 days (24). However said drug and said method of prophylaxis and treatment using this drug are insufficiently universal.

The present medical compositions for the use in medicine comprise peptides corresponding to the below general formulae and forming the family of delta-sleep peptide (FDSIP). FDSIP peptides include DSIP and also a group of its analogues and derivatives containing 4-9 or more amino acid residues:

[I] X-Y¹-Y²-Y³-Y⁴-R

[II] X-Y¹-Y²-Y³-Y⁴-Y⁵-R

[III] X-Y¹-Y²-Y³-Y⁴-Y⁵-Y⁶-R

[IV] X-Y¹-Y²-Y³-Y⁴-Y⁵-Y⁶-Y⁷-R

[V] X-Y¹-Y²-Y³-Y⁴-Y⁵-Y⁶-Y⁷-Y⁸-R

[VI] X-Y¹-Y²-Y³-Y⁴-Y⁵-Y⁶-Y⁷-Y⁸-Y⁹-R,

where
X = H or acyl residue of formic, acetic, propionic or an other fatty acid, and also their aryl derivative,
Y¹ = Trp or Tyr residue of L- or D-configuration or corresponding N-methyl derivative,
Y² = Ala, N-Me-Ala, β-Ala, Gly, Pro, Tyr, Ser or hydroxy-proline residue of L- or D-configuration,
Y³ = Gly or Ala, N-Me-Ala, Pro or hydroxy-proline residue of L- or D-configuration,
Y⁴ = Gly or Ala, N-Me-Ala, Pro or hydroxy-proline residue of L- or D-configuration,
Y⁵ = Asp, Glu residue or corresponding N-methyl derivative of L- or D-configuration,
Y⁶ = Ala residue or other protein amino acid or hydroxy-proline residue of L- or D-configuration or corresponding N-methyl derivative,
Y⁷ = Ser residue or other protein amino acid or hydroxy-proline residue of L- or D-configuration or corresponding N-methyl derivative,
Y⁸ = Gly residue or other protein amino acid or hydroxy-proline residue of L- or D-configuration or corresponding N-methyl derivative,
Y⁹ = Glu residue or other protein amino acid or hydroxy-proline residue of L- or D-configuration or corresponding N-methyl derivative,
R = -OH, -OR₁, -NH-R₁, (where R₁ = H or -alkyl, arylalkyl).

Moreover, delta-sleep peptide family includes peptides [I-IV] extended at the N- or C-terminus by polypeptide chains composed of 1 - 9 protein amino acid residues, wherein N- and C- terminal residues of the extended peptides may be free or may be blocked by X and/or R groups. Delta-sleep peptide family includes also multimeres of family peptides and peptides associated with different carriers (for example, polyethylene glycol).
The present medical compositions comprise the abovementioned peptide derivatives and one or more other synergistically acting components , such as various protein amino acids (for example, inhibitory neurotransmitter glycine, arginine and other amino acids), natural direct antioxidants, such as carnitine, carnosine, homocarnosine or their precursors being the corresponding acetyl derivatives. The present compositions may also comprise various other neuroprotective metabolically active components (one or more), like phospholipids (lecithin), unsaturated fatty acids including ω-3-fatty acid derivatives, choline and its derivatives, trimethylglycine, , vitamin C, vitamin A and / or its precursors, nicotine amide (vitamin PP), vitamin E (α-tocopherol), vitamins of the B group (B₁, B₆, B₁₂, B₃), folic acid, α-lipoic acid, eicosapentaenoic acids and docosahexaenoic acids, plant bioflavonoids, coenzyme Q10, taurine, terpenes, polyphenols, plant neutriceuticals on the basis of extracts (from garlic, onion, green tea, ginseng, grapes, licorice, ginger, *Gotu kola, gingko biloba,* coniferous plants), succinic acid, *Riboxinum,* macro- and trace elements (magnesium, potassium, calcium, chrome, selenium ions, etc.) etc. The compositions may comprise other acceptable ingredients, commonly used as fillers, taste improving and flavoring agents, sweeteners, conservatives, etc. for the preparation of drug products. The present compositions based on the natural components can also be comprised in parapharmaceutical agents, biologically active additives, homeopathic agents and infant's food.

The agent is characterized in that the compositions may comprise also other ingredients commonly used for the preparation of drug products, in particular bestatin or other inhibitors of aminopeptidases, which can also be comprised in parapharmaceutical agents, biologically active additives, homeopathic agents and infant's food.

The weight ratio of the main active components of the composition varies in the following range: peptide component : amino acid component : natural antioxidant = 1 : (0-250) : (0-250).

The present compositions can be administered intranasally in form of nose drops or in form of a spray; as vaporized powder, as an aerosol, sublingually in form of resorbable tablets, in form of capsules and powders; in form of different injections (intravenous, intramuscular, subcutaneous and intracutaneous); and also as suppositories, ointments, creams, lotions, and can also be incorporated into products for children's nutrition.

Listed below are main fields of use of the present invention together with clinical examples of the use of the agent for the correction of neurotransmitter, neuroendocrinal and metabolic disorders occurring due to various stress factors.
1. Efficacy of use with respect to diseases / conditions caused by emotional, physical, psychological or pathophysiological stress factors:
   1.1 The preparation is effective in the case of emotional and mental stress, i.e. during exam or test preparation. Intranasal administration at a dose of at least 0.3 mg at least two times per day for at least 2 days to adults (to children depending on age: 1 - 2 ampoules per day for 10 days) significantly increases the capability for learning large amounts of information and reduces the time needed for material repetition during exam preparation and analogous situations. In the case of rest necessity at least a single application of the preparation provides a satisfactory feeling of rest and recreation.
   1.2 The preparation is effective in the case of significant emotional and physical stress, i.e. during long-distance passages and long-distance flights, in the case of frequent changes of time zones and in the case of frequent changes of the communication language. Intranasal (sublingual) administration in a dose of at least 0.3 mg one - two times per day allows easy / painless overcoming of emotional and physical stress associated with traveling.
   1.3 The preparation is effective in the case of significant psychological and physical stress, i.e. sport competitions on a professional and/or amateur level and intensive training. Intranasal administration in a dose of at least 0.3 mg one time per day for at least 5 days with repeating courses allows tolerating intensive physical stress without psychological breakdowns.
   1.4 When using the preparation to prevent and to reduce manifestations of acute stress conditions and symptoms of exhausting stress overstrain in a dose of at least 0.3 mg daily in case of a borderline neuropsychic pathology an overcoming of insomnia, a reduction of time for testing tasks completion and an increase of resistance and of the long-term attention stability occur.
   1.5 The use of the preparation in extreme conditions of military operations led to a massive reduction of complications of neuropsychic character and also to an acceleration of the processes of wound healing and rehabilitation including those after firearm wounds and fractures. At intranasal administration of at least one ampoule of the preparation per day in conditions of exposure to extreme factors no evidence of insufficiency of vegetative neural systemlike tachycardia development, elevation of systolic and/or diastolic pressure has been found. Moreover, a decrease of anxiety and psychic tension, headache, dizziness and tympanophonia has been observed.
   1.6 The preparation is effective under exposure to pathophysiological stress factors, i.e. for reducing the withdrawal syndrome in therapy of opioid addiction. Intravenous administration in a dose of at least 0.3 mg at least 4 times per day for at least 3 days led in 97% of the cases to a rapid disappearance of clinical manifestations of withdrawal symptoms. Intranasal administration in a dose of at least 0.3 mg per day for at least 3 days provides significant influence on somato-vegetative symptoms and is effective in complex treatment of opioid withdrawal syndrome.
   1.7 The use of the preparation is highly effective in the case of long-term stress disorders as to correct neurotic disorders, including reduction of neurotic manifestations in case of neurasthenia with anxious-phobic disorders, alcoholism and opioid addiction without simultaneous use of other drugs. The course dose is at least 60 mg (6 mg per day for 10 days).
   1.8 As to reducing ethanol addiction and the withdrawal syndrome of associated forms of alcoholism. Administration in a dose of at least 3 mg/day resulted in 72% of the cases in a reduction of vegeto-asthenic disorders compared to the control group and in a significant sleep improvement. In a further course of treatment the preparation demonstrated a significant influence on primary alcoholic attraction. The time of remission in patients receiving Deltaran far exceeded the control group: maximum time was 18 month and 1 month, respectively. It is particular importance that in the case of a failure the narcotic intake was not accompanied by common euphoria occurrence that can be considered as restoration of normal sensitivity of opioid receptors in patients receiving Deltaran.
   1.9 Course administration of the preparation leads to an increase of potency in case of functional disorders of sexual activity. Long-term use of small doses of the drug (at least 3 mg per day for at least 2 weeks, repeated at least 2 times a year) allows durable preserving the sexual activity in particular of elderly people and under conditions of chronic stress.
2. Use of the drug with respect to different manifestations of the metabolic syndrome is effective in case of repeated course administration each of at least 5 days.
   2.1 Use of the preparation in the case of the cardiovascular system pathology. The drug provides a stabilizing action on the contractile myocardial function and on the electrical activity. Intranasal administration of at least 1 - 2 ampoules for at least 7 days to patients with a severe progressing course of ischemic heart disease against a background of hypertonic disease of grade II-III led to a stabilization of the condition, to the termination of heard strokes and to the termination of extra systoles and ECG-signs of transient myocardial ischemia. Course administration of the drug resulted in easy tolerance of coronary angiographic procedures and led to the possibility of reducing the dose of long-term administered preparations. A drug monotherapy in elderly patients with ischemic heart disease (older than 70 years) led to a significant reduction of complaints associated with multiorgan pathology and metabolic syndrome, and thus to an increase in life quality of the patients. Intranasal course administration of the drug during at least the first 5 - 10 days of each month increases the resistance of elderly patients to the physical and emotional stress, reduces meteosensitivity and the need for coardiotropic drugs.
   2.2 Efficacy of ambulant use of the preparation in the therapy of elderly patients with diabetes mellitus of type II. Intranasal course administration of the drug in a dose of at least 3 mg per day for 20 days led to a subjective improvement of life quality (improvement of short-term memory, reduction of depression, anxiety, hidrosis, and also hunger tolerance). A significant reduction of the intensity of diabetic neuropathy manifested itself in an increase of the vibration sensitivity level, in the disappearance of convulsions, paresthesias and numbness of lower extremities. The majority of patients showed a decrease of systolic and diastolic pressure and also an improvement of the glycemic profile values. The drug shows a normalizing effect on age-associated hormonal-metabolic processes and is a valuable additive to sugar-decreasing therapy in patients with a geriatric profile.
   2.3 The use of the drug is safe and effective in middle aged patients suffering from diabetes mellitus type II of a moderate severity. A course of treatment of at least 3 mg per day two times per day during first six days, then 3 mg once per day in the morning for at least 8 days after achieving satisfactory compensation of diabetes mellitus leads to an increase in the portion of patients with a good compensation of diabetes mellitus A significant reduction of daily glucosuria has been observed. The therapy provides a positive effect on arterial pressure, on the carbohydrate metabolism and on the functions of the vegetative nervous system.
   2.4 The use of the drug is effective to reduce neurocirculatory dystonia and discirculatory encephalopathy of atherosclerotic genesis in case of degradation of the memory and of mental working capacity. In these kinds of pathology a regular application of a course of treatment is necessary of at least 3 mg per day during first 5 - 10 days of each month, and subsequently a similar treatment course every three months and thereafter every 6 months, but not less frequently. Such treatment courses of discirculatory encephalopathy of atherosclerotic genesis lead to a reduction of insomnic and cephalgic syndromes, of dyspraxia, vestibular, ataxic and cochlear disorders, as well as to a definite improvement of the function of optico-dimensional gnosis and of the audio-verbal memory, which generally leads to an increase of life quality of said category of patients.
   2.5 In the case of incorporation of the drug into a therapy of vegetative vascular dystonia, an improvement of sleep quality is achieved as well as a reduction of encephalic syndrome, a significant decrease in frequency of migraine attacks, a decrease of anxiety, irritability and mood fluctuations. During treatment a significant decrease in frequency of vegetal crises and their intensity in 30% of patients occurs and a termination of crises occurs in 70% of patients. The duration of the treatment effect is preserved during monotherapy with at least 3 mg/day for at least 10 days, a subsequent two-week interruption and repeated course of treatment for 3 - 5 months.
   2.6 In the case of incorporation of the drug into a therapy of patients suffering from migraine, a change of the vegetative index is observed that is shifted towards the zero value, thus achieving vegetative balance. An adaptation to physical stress occurs. During the first half of the year after the conduction of drug therapy with a dose of at least 3 mg/day for at least 7 days the frequency, the extent and the duration of migraine attacks decrease significantly.
   2.7 The use of the drug in patients with moderate and severe bronchial asthma by at least 6 mg per day for five days, then 3 mg per day (in the evening) for 20 days leads to a significant reduction of the need for use of beta-agonists in patients by way of reducing bronchial obstruction. The drug potentiates the action of short-term acting beta-agonists and allows a significant reduction of their maintaining doses. The use of the drug reduces the variation of daily average values of peak expiratory rate, thus improving the controllability bronchial asthma.
   2.8 Pharmacological efficacy of the drug was proven with respect to acute pancreatitis. An intranasal or intravenous administration of the drug in a dose of at least 6 mg per day for 5 days leads to a significantly decrease of the content of non-peptide components of medium weight molecules that play a considerable role in the development of endotoxicosis.
3. Use of the preparation with respect to pathological conditions caused by different infectious and/or autoimmune agents.
   3.1 The use of the preparation in neuroborreliosis. Intranasal administration in the course dose of at least 6 mg/day for 5 days and subsequently of3 mg per day (in the evening) for 20 days (60 mg, 20 ampoules) leads to the disappearance of complaints regarding irritability, sweating, headache and sleep disturbance. One month after the start of the therapy a positive dynamics of neurological symptoms is obtained in 70% of the patients. Immunological evidence of the absence of destructive processes in neural tissue is achieved 3 - 6 months after starting the therapy using the drug.
   3.2 Use in multiple sclerosis. Incorporation of the drug into a complex therapy of multiple sclerosis in a dose of at least 6 mg per day for 5 days with a subsequent administration of 3 mg per day (at the evening) for 20 days led to a significant reduction of vegetative dystonia and intellectual-mental disorders. The intensity of the positive drug effect is directly proportional to the level of irregularity of sleep structure and of psychoemotional condition. a positive dynamics in the emotional sphere was achieved in 75.6% of the patients and a reduction of the syndromes of vegetative and neurocirculatory dystonia of the hypotonic type was achieved in 83.3% of the patients by the use of the drug for 10 days.
   3.3 Use in autoimmune thyrioiditis. Intranasal administration in the dose of at least 3 mg for at least 10 days led to a decrease in the thyroid tissue antibody concentration. Furthermore, a trend to normalization of thyroid hormone levels was observed.
4. Incorporation of the drug into the course of treatment of patients with infantile cerebral paralysis (such spastic forms like spastic diplegia, hemiparetic form, atonic-astatic form, consequences of cranio-cerebral trauma) led to a reduction of muscular tonicity, to an expansion of range of motions and to an increase of muscular strength. A positive effect was achieved when the drug was administered intranasally in a dose of at least 0.3 mg for at least five days.
   4.1. In the case of incorporation of the drug into a complex program of rehabilitation of children with minimal brain dysfunction and with functional and organic abnormalities of the central nervous system function in a dose of at least 3 mg for at least 10 days led to an improvement of intellectual-mnestic functions, an improvement of psycho-emotional condition and to an improvement of sleep. Definite reorganization of neurodynamics and bioelectrical brain activity is achieved. A normalizing effect on the values of alpha-activity indicates an improvement of the interaction of inter-structural and inter-centre relationships in the damaged brain. The drug provides positive treatment effects in the case of its incorporation into the products for children's nutrition.
   4.2. In the case of clinical manifestations of perinatal pathology of newborn children (abundant regurgitation within one hour and more after feeding, head-tilt in sleep, increased pulsation of anterior fontanel, muscular hypotonicity or hypertonicity, superficial sleep, day and night confusion) 5 - 10 ampoules of the drug are intranasally administered to the mother in the case of breast-feeding. Intranasal administration of a dose of at least 3 mg for 5 - 10 days (bottle-fed children were administered from ½ ampoule (1, 5 mg) intranasally for at least 10 - 14 days) led to a significant positive dynamics. Repeated conduction of course therapy significantly improved values of physical, psychic and emotional development of the children.
   4.3. In the case of incorporation of the drug into a therapy of patients suffering from symptomatic and idiopathic epilepsy a decrease in frequency of the development of attacks or a stopping of said attacks occurs. A positive effect is observed by a course administration of at least 6 mg per day for 20 - 30 days. A repetition of analogous or shorter courses of therapy led to a significant prolongation of the free period.
   4.4. Use of the preparation in pregnant women actively taking heroin. The manifestations of the abstinence syndrome were rapidly reduced in pregnant women, who terminated the narcotic intake and used Deltaran in a dose of at least 6 mg per day for 20 - 30 days during pregnancy and/or prepartum. The newborn children did either not develop an abstinence syndrome or developed an abstinence syndrome in a form, which did not required any treatment.
5. Possibilities of using the drug in oncology practice. Incorporation of the drug into a complex concomitant therapy of oncologic diseases leads to an improvement of psycho-emotional condition of the patients, to an increase of motion activity, to an improvement of sleep and appetite, and an increase of the pain limit. An intranasal administration of the drug in a dose of at least 0.3 mg per day for 7 - 10 days allows a decrease of the necessary dose of narcotic and non-narcotic analgetics in case of difficulties in or even impossibility of radical treatment of the disease. In the following conduction of short-term courses of at least 2 - 3 days and a period between the courses of 7 - 10 days is effective.
   5.1. The use of the drug in a minimal dosage of at least 3 mg/day for at least 5 days at the early period of polychemotherapy-induced pancytopenia led to a more rapid restoration of the leucocytes and consequently to a lower frequency of infectious complications and therefore to a reduced need to antibacterial and antimycotic therapy. The described effectiveness of the drug is realized in the treatment of children as well as of adult oncology cases. The average duration of leucopenia in patients receiving the drug was 8.2 ± 0.5 days, and in patients that did not receive the drug aid leucopenia lasted for 13.2 ± 0.8 days. A significant improvement of the psycho-emotional status made it easier for children with an oncologic disease to stay in the hospital.
6. Use of the preparation in a complex therapy of burnings in pediatric practice. Intranasal administration of the drug in a daily dose of at least 3 - 9 mg led to an acute acceleration of the recovery from shock condition and subsequently, when continuing the therapy in the post-shock period to a significant improvement in psycho-emotional sphere (sleep improvement, appetite improvement, reduction of bandage fear), and to an acceleration of healing and graft acceptance in the treatment of children with a damage area of more than 47% of the body surface and a marked septic toxemia.
   6.1. Intranasal administration of the drug in a dose of at least 3 - 6 mg per day leads to a significant acceleration of wound surface healing and to an improvement of the psycho-emotional state of the patients in the case of a significantly lower damage.
7. Use of the preparation in complex therapy of severe cranio-cerebral trauma. Intranasal administration of high doses of the preparation (up to 15 mg per day) allowed to save life of patients that underwent a life-threatening severe cranio-cerebral trauma. In the case of a long-term control of patients after a severe cranio-cerebral trauma with a surgical removal of the crush injury locus of the brain a course administration of the drug in a dose of at least 3 mg twice per day for 5 - 7 days led to the disappearance of pathological phenomena in 70% of patients during the first month, and to a normalization of sleep in 100% of patients. Functional stress did not cause paroxysmal activity in the majority of patients, which allowed in 24% of the patients to reduce the dose of anticonvulsive drugs and in 4% of the observation to cancel these drugs totally.
   7.1. In the case of incorporation of the drug into a complex therapy of patients with a history of diffuse axonal damage in conditions of deep coma, where a stable vegetative condition is not formed, a regress of neurological deficit in motion and sensuous spheres occurs and patients can attend to themselves. Intranasal or intravenous drug administration in a dose of at least 3 mg/day for at least one week to treat such severe patients allows to reduce lethality (more than two fold), to improve prognosis for conscious restoration, to significantly reduce invalidization and to improve quality of life. In the case of use of the drug the expenses for conventionally used drugs are significantly reduced.
   7.2. Use of the drug is effective and justified in the case of pathology of central and peripheral nervous system (meningitis, encephalitis of any genesis, paresis, and insults). In the case of emergency situations (shock of any genesis) the use at least 3 ampoules of the preparation per day until recovery from the critical condition leads to a significant reduction of lethality, to an improvement of the subsequent course of the disease and to a significant reduction of the hospitalization period.
8. The use of the drug in surgery is indicated for acceleration of wound healing, for creating conditions of primary adhesion of postoperative wound and for avoiding formation of deep scars and keloids. Multiplicity of the metabolic effects of the drug and its anti-stress activity *inter alia* provide for a reduction of immune depression and therefore lead to an acceleration of wound healing when administered intranasally in a dose of at least 3 mg per day during the entire postoperative period.
   8.1 Prophylactic intranasal administration of the drug in a dose of at least 3 mg per day 5 days before operative treatment in case of planned operations significantly reduces the risk of post-surgical complications and significantly increases the rate of wound healing, even in gerontological patients. In the case of drug use in the pre- and post-surgical period the wound practically always heals by first intention.
   8.2 Course use of the drug in the pre-surgical periods in case of a planned operation with an artificial blood circulation provides for an advantageous course of narcosis recovery and for a significantly reduction of the risk of severe post-operative complications and for a reduction of the hospitalization duration. The intranasal or intravenous drug administration in a dose of at least 3 mg per day during the entire pre- and postoperative period is highly effective for correction of insomnia and asthenic conditions in pre- and post-surgical periods (for extensive surgical interventions including those using artificial blood circulation).
   8.3 Use of the drug in patients with pyoinflammatory diseases of the maxillofacial region, including those with severe background pathology, leads to a decrease of the signs of intoxication, edema, functional impairment and pain syndrome. An intranasal course administration of the preparation in a dose of at least 3 mg per day during the entire acute phase of a purulent inflammation leads to a positive effect with respect to wound cleansing, to generation of primary granulation and to enhancement of the antibacterial effect of systemic and local antibiotics.
9. Intranasal application of the drug in cosmetic medicine leads to a substantial improvement of the skin turgor, to an improvement of microcirculation, to the ability of the skin to conserve moisture and to an improvement of the resistance to common infections. A regular conduction (not less than once in a month) of short-term treatment courses with at least 3 mg per day for 5 - 7 days during a long-term period (of half a year and more) is effective in cosmetic medicine. Incorporation of the drug into the composition of creams, including creams for the skin treatment around the eyes, of tonics, lotions and milk for skin treatment of the face and the body in a dose of at least 3 mg per volume of the cosmetic for one skin treatment application considerably improves the microcirculation and a consequence also improves the complexion, leads to the disappearance of dark circles under the eyes, and normalizes skaling of affected skin at the face and the trunk in a course treatment for at least one week. Incorporation of the drug into the composition of creams, including creams for the skin treatment around the eyes, of tonics, lotions and milk for the skin treatment of the face and the body in a dose of at least 6 mg per volume of the cosmetic for one skin treatment application leads to a significant improvement of the status of the facial skin and the periorbital region for a significant time period.
10. The use of the preparation in veterinarian medicine regarding small, middle and large mammals, including dogs and cats allows for a significant reduction of the treatment period of contagious and parasitic diseases and for an increase of the resistance to various infectious contaminations and stressful situations (e.g. exhibitions, passages, changes of owners, etc.) by intranasal or intramuscularly, subcutaneous or intravenous administration in a dose of at least 1 mg for 1 - 5 days.
11. Application of the drug in combination with the synergistically acting amino acid glycine being an inhibitory neurotransmitter and its derivative trimethylglycine allows for the use of the agent in shorter treatment courses of at least 1 day, achieving a similar and even a greater positive therapeutic effect.
12. The use of the agent in combination with such biogenic matter like carnitine, carnosine, homocarnosine or their precursors in intranasal, intramuscular or intravenous application in a dose of at least 3 - 6 mg at least 1 - 2 times per day for at least 5 - 10 days results in improvement of the appetite, in growth acceleration and body weight gain and supports normalization of the hyperthyroidism metabolism. Furthermore, said combined drug use is effective in the treatment of anorexia caused by nervous and physical attrition or after previous diseases, operations, and in chronic ischemic heart disease.
13. Use of the agent in combination with Nicotinamide or vitamin PP (niacin) representing a hydrogen carrier and effecting oxidation-reduction-processes supports an improvement of the carbohydrate metabolism particularly in case of diabetes mellitus type II in middle-aged and elderly patients (by intranasal or intravenous application in a dose of at least 3 - 6 mg at least 1 - 2 times per day for at least 10 - 20 days), and is also indicated in case of liver diseases, and peptic ulcer of the stomach and the duodenum (by intranasal or intravenous application in a dose of at least 3 mg at least 1 - 2 times per day for at least 5 days), in case of slow-healing ulcers and wounds including those of trophic nature in varicose veins of the inferior extremities (by intranasal or intramuscular application in a dose of at least 3 mg at least 1 - 2 times per day for at least 5 days, and also by local treatment of the damaged surface in a dose of at least 6 mg per volume of the agent for ligation).
14. Use of the agent in combination with vitamin B₁ (Thiamine) in neuritis, radiculitis, neuralgia and peripheral paralysis supports a faster resolution of the acute symptoms, a reduction of the hospitalization period and a reduction of invalidism by intranasal or intramuscular application in a dose of at least 3 mg at least 1 - 2 times per day for at least 5 - 10 days.
15. Use of the agent in combination with vitamin B₁ (Thiamine) and vitamin B₁₂ (cyancobalamin) and their derivatives in dermatology is effective in resolution of neurogenic pruritus, is effective in pyoderma, psoriasis or eczema by intranasal, intravenous or intramuscular application in a dose of at least 3 mg at least 1 - 2 times per day for at least 5 -10 days.
16. Use of the agent in combination with Vitamin B₆ (pyridoxine) and its derivatives supports an enhancement of the metabolism of tryptophane, methionine, cystein, glutamic and other amino acids and supports the resolution of pregnancy toxemia and improving the condition of the fetus in water rich pregnancy by intranasal or intramuscular application in a dose of at least 3 mg at least 1 - 2 times per day for at least 5 - 10 days.
17. Use of the agent in combination with Vitamin B₆ (pyridoxine) and its derivatives supports an increase of the efficacy of recombinant growth factors in leukopenic conditions and allows for a reduction of their course of application by intranasal, intramuscular or intravenous application of the agent in a dose of at least 3 - 6 mg at least 1 - 2 times per day for at least 5 - 10 days.
18. Use of the agent in combination with Vitamin B₆ (pyridoxine) and its derivatives allows for an application of the agent at minimal doses obtaining analogous and even higher positive therapeutic effect in Ménière's disease, in sea and air sickness and also in involutional depressions by intranasal application of the agent in a dose of at least 3 - 6 mg at least 1 - 2 times per day for at least 10 - 20 days.
19. Injections of the agent in combination with vitamin B₁₂ (cyancobalamine) or its derivatives is indicated for the reduction of painful syndrome in treatment of diseases of the central nervous system, of traumatic diseases of peripheral nerves, for the reduction of the frequency of migraine attacks by intramuscular or intravenous administration of the agent in a dose of at least 3 mg at least 1 - 2 times per day until reduction of the painful syndrome or for at least 5 days each month.
20. Use of the agent in combination with phosphatide lecithine and its constituent choline reduces or prevents fatty liver infiltration and is indicated for Botkin's disease, hepatitis, liver cirrhosis (mainly in early stages), hypothyroidism, cystinuria, and chronic alcoholism by intranasal or intramuscular application of the agent in a dose of at least 3 mg at least 1 - 2 times per day for at least 10 days with repeated courses of at least two times a year.
21. Intranasal or intramuscular application of the agent in combination with lipoic acid in a dose of at least 3 mg at least 1 - 2 times per day for at least 10 days with repeated courses of at least 3 times a year is indicated for the prevention and treatment of coronary atherosclerosis, liver diseases (light or middle-severe Botkin's disease - in absence of an intensive or increasing jaundice; chronic hepatitis, cirrhosis), of diabetic polyneuritis, and of intoxications.
22. Intranasal application of the agent in combination with ascorbic acid in a dose of at least 3 mg at least 1 - 2 times per day for at least 5 days is indicated for prophylaxis and treatment of Barlow's disease, of hemorrhagic diathesis, of nasal, parenteral, pulmonary and other hemorrhages, and also of an overdosage of anticoagulants, of contagious diseases, of slow-healing wounds and fractures of bones. Use of the agent in combination with ascorbic acid improves the tolerance to serious exercise stress and strain, particularly in pregnancy.
23. The long-term use of the agent in combination with the vitamin E (alpha-Tocopherol) reduces the severity of clinical manifestations and increases the duration and stability of neurologic remission in neuromuscular diseases (e.g. muscular dystrophy, amyotrophic lateral sclerosis, etc.).
24. Short-term intensive intranasal application of the agent in a dose of at least 6 mg at least 1 - 2 times per day for at least 5 days in combination with the vitamin E (alpha-Tocopherol) to men suffering from a disturbance of spermatogenesis and potency results in recovery of normal erectile function.
25. Use of the agent in combination with vitamin A (Retinol) and/or its precursors in a dose of at least 3 mg at least 1 - 2 times per day for at least 10 days allows for a reduction of the volume of therapeutic interventions in skin damages and skin diseases (e.g. chilblains, wounds, ichthyosis, follicular dyskeratosis, senile keratosis).
26. Course application of the agent in combination with Riboxinum (a derivative of the nucleoside purine) in a dose of at least 3 mg at least 1 - 2 times per day for at least 10 days allows for a reduction of the volume of therapeutic interventions in ischemic heart diseases (e.g. chronic coronary failure and myocardial infarction) and in myocardial dystrophy.
27. Course application of the agent in combination with vegetable bioflavonoides being capable of stimulating and accelerating tissue reparation processes allows for an effective use of the agent in repeating short-term administration courses in a dose of at least 3 mg within one - three days with intervals between the courses of up to 4 weeks, thus creating conditions for a significant improvement of the metabolism of tissues exposed to oxidative stress.
28. Intranasal, intramuscular or intravenous repeated use of the agent in combination with succinic acid and its derivatives, and also with macroelements and trace elements (e.g. magnesium, potassium, calcium, chrome, selenium ions, etc.) in a dose of at least 3 mg within 3 - 5 days with intervals of up to 4 weeks and a repetition of courses at least once in three months shows a significant positive effect on liver metabolism, and is indicated for acute and/or chronic intoxication (including alcoholic intoxication) and for functional disorders of the liver caused by hepatitis. Use of the agent in combination with succinic acid and derivatives thereof considerably enhances the tolerability of intensive physical stress, increases the resistance to air pollution by toxic waste products (including conditions of living in a megacity), and supports the conservation and maintenance of mnestic functions under conditions of sustained attention.
29. Course application of the agent in combination with herbal nutriceuticals on the basis of extracts of garlic, onion, green tea, ginseng, grapes, licorice, ginger, *gotu* kola, *ginkgo biloba* and coniferous plants. in a dose of at least 3 - 6 mg per day with a ratio of 3 mg agent : 10 - 250 therapeutic doses of the nutriceutical at least once per day for at least 10 - 15 days leads to a significant improvement of the quality of life, to an improvement of tolerance to psycho-emotional, physical and infectious-induced stress, and is indicated for prophylaxis of development of stress-associated diseases and for the treatment of various manifestations of the metabolic syndrome.
30. The use of the preparation is also indicated for other cases, including: for prophylaxis and treatment of sea and air sickness, wherein intranasal or intramuscular application of the agent according to claim 1 or claim 2, or claim 3, or claim 4, or claim 5, or claim 6, or claim 7, or claim 8 is performed in a dose of at least 3 mg at least 1 - 2 times per day for at least 1 - 2 days in combination with Vitamin B₆ (pyridoxine) and its derivatives.

The use of the drug does not provide a direct sedative effect. The intensity of the somnolescent effect of the preparation is directly proportional to the level of abnormalities in the sleep structure and the psycho-emotional condition.

According to its molecular structure and its chemical and biological effect the drug is phylogenetically conservative and not species-specific and therefore not able to cause an allergic reaction. In the clinical use no case of intolerance of the drug was observed.

The preparation compensates a DSIP-deficit of the organism providing for a restoration of the central nervous system work and optimizes its function. The drug therefore consequently leads to a favorable course of diseases in terms of highly increasing efficacy of standard medical agents and preventing the development of complications. The multiplicity of the effects of the drug is associated with the manifestation of the central DSIP effect being the normalization of the cell respiration function by acting on the cellular mitochondrial apparatus by modification of the GABA-receptor activity.

The stabilization of CNS functions and the neuroprotective activity (prevention of neuronal loss) according to the present invention allow to use of the present agent as follows:
1. in emergency medicine for reducing acute, life threatening conditions;
2. in preventive medicine, even under arctic conditions, emergency conditions and conditions of technogenic catastrophe, for the prevention of development of complications in terms of physical and psycho-emotional overloads, for the decrease of central nervous system disorders in different manifestations of the metabolic syndrome. A single course and/or permanent application of the agent provides for an optimal level of performance capability of operators under extreme conditions (intensive physical and psycho-emotional stress, exposure to unfavorable environmental factors - including climatic, toxic, etc.);
3. for prophylaxis and improvement of efficacy of treatment of diseases the severity of which is stress-associated to a certain extent, such as cardiovascular diseases (infarction and insults), diabetes, etc.;
4. in the treatment of infectious diseases and diseases complicated by secondary infections: for reduction of manifestations of secondary immunosuppression to provide a significant improvement of the etiopathology of such diseases like tuberculosis, Lyme disease, pneumonia, wound infections, etc. Single - twofold application of the drug prior to conduction of a prophylactic vaccination significantly reduces the risk of development of complications in infant or adult patients with a burdened anamnesis.
5. in restorative medicine and operative surgery for the normalization of the regenerative processes after trauma, after chemical intoxication and operative treatments. Course application of the drug in pre- and postoperative period in case of planned operations, including operations with application of extracorporeal circulation prevents development of post-operative complications and is indicated for preventing of development and for treating posttraumatic stress disorder;
6. in cosmetic medicine: the long-term use of short-term courses of the drug improves significantly the skin turgor, the microcirculation, the resistance to common infections, and skin's ability to restore moisture;
7. the use of the preparation retards development of neurodegenerative processes in the CNS in case of chronic and acute intoxication (in particular alcoholic and narcotic intoxication or of other diseases (multiple sclerosis, gout, senile dementia) and also decreases the extent of vascular and metabolic abnormalities, associated with the aging process. The agent is effective in the prevention of neurodegenerative processes in patients with alcoholic and other neurotropics and narcotics abuse, and in the treatment of cranio-cerebral trauma and ischemic brain damage. The agent is also an anti-aging drug;
8. in reproductive medicine and neonatology for significant reduction of manifestations resulting from birth traumas (infantile cerebral paralysis, etc.), of traumatic brain injury (TBI), of unfavorable influence on the fetus by metabolic and toxic pathologies of the mother (children of narcotic addicts, children's diabetes, late toxicosis). The drug may be used as a agent for fetal protection under conditions of unfavorable effects induced by metabolic, traumatic, toxic and other factors;
9. in pediatrics for compensation of an endogenous FDSIP deficiency in the early postnatal period in children not receiving breast milk, and also for normalization of psychic health of children suffering from hyperexcitability and attention deficit syndromes;
10.repeated course administration of the preparation is indicated for prophylaxis of development of malignant neoplasms under apparent effect of cancerogens and procancerogens of various acting mechanism (in particular under conditions of unhealthy and hazardous production, technogenic accidents, etc.). The antimutagenic effect of the preparation is due to its basic acting mechanism in particular under the massive impact of mutagens (ionizing radiation, radiotherapy and X-ray therapy, multiple repeated X-ray examinations, etc.);
11. repeated (multiple) long-term (not less than 21 - 30 days) course administration of the preparation with a minimal course dose of not less than 60 mg leads to a significant reduction in frequency of the development of convulsive attacks in epilepsy, to a normalization of activation-inhibition processes, to an improvement of mnestic functions and to a significant reduction of dyssomnia;
12.The method has been approved for treatment of animals in case of infectious, parasitic, noncontagious internal and surgical disorders to allow for a reduction of the period of traditional treatment by at least 2 times, to allow for preventing complications and massive reducing the number of fatal cases by administering the drug, wherein intranasal or subcutaneous administration is performed in a dose of at least 1 - 3 mg per day for at least 2 - 5 days. The use of the drug leads to an acceleration of regenerative processes in affected organs and tissues and to acceleration of recovery.

The method of prophylaxis and treatment of stress conditions using the present agent is characterized in that the compositions according to the invention are used:
- intranasally in form of nose drops or in form of a spray;
- sublingually in form of dissolving tablets or capsules;
- in form of powders, suppositories, ointments, creams;
- in form of different injections (intracutaneously, subcutaneously, intramuscularly, intravenously);
- in form of different cosmetics (creams, lotions, tonics, cosmetic milks, foams, soaps, etc.);
- as components of the products for children nutrition.

### References

1. Bogolepov N.N., Popova E.N., Koplik E.V., Krivitskaya G.N., Sudakov K.V. Structural-functional organization of neurons in the cerebral cortex of rats with different levels of resistance to emotional stress in conditions of exposure to delta sleep-inducing peptide // Neurosci Behav Physiol. 2004 - V.34 (6) - p.611-616.
2. Rihireva G.T., Makletsova M.G., Mendzheritsky A.M., Vartanjan H.p., Gurevich S.M., Lozovskaja E.L., Kopylovskij S.A., Rylova A.V., Mihaleva I.I., Prudchenko I.A. Modification of intensity of free-radical reactions in organs of rats in hypokinetic stress and protection delta sleep by an inducing peptide and its tyrosin-including clone. // Bulletin of the Russian Academy of Science 1993. - vol. 2. - p.243-256.
3. Howell T.H. Neurological problems of the elderly // Gerontology Clin. (Basel) 1966.-Vol.8.-N. - p.77-94.
4. Seidel G., Adermann K., Schindler T., Ejchart A., JaenickeR., Forssmann W.-G., Rösch P. Solution Structure of Porcine Delta Sleep-inducing Peptide Immunoreactive Peptide A Homolog of the Shortsighted // Gene Product 1997. - Vol. 272, N. 49, p. 30918-30927.
5. Najimi M, Bennis M., Moyses E., Chigr F. Distribution of delta sleep-inducing peptide in the newborn and infant human hypothalamus: an immunohistochemical study // Biol. Res. - 2001. - V.34. - n.1.
6. Macionis A.E. Morphometric mechanisms of synaptic plasticity and their role in adaptation of brain to extreme states. The autoabstract of a thesis for a degree of doctor of medical sciences 1997. p.1-40.
7. Alperovich D.V., Lysjenko A.V., Matsionis A.E., Mendzheritsky A.M. Biochemical mechanisms underlying neuroprotective effect of delta-sleep inducing peptide under hypoxia // Hyp. Med. J. - 1997. V.5 (4.) - p. 3-7.
8. Zoolan («Deltacin») clinical application in a veterinary medicine (treatment of dogs and cats). Methodical references. - V.P.Urban, B.O.Vojtenkov, N.P.Batsanov, M.M.Shirobokova, G.M.Gromov, I.A.Tishko, S.V.Valeeva's Makers. - SPb. - 1999. - 10 pages.
9. Khvatova E.M., Samartzev V.N., Zagoskin P.P., Prudchenko I.A., Mikhaleva I.I. Delta sleep inducing peptide (DSIP): effect on respiration activity in rat brain mitochondria and stress protective potency under experimental hypoxia // Peptides 2001. - Vol.24. - p.307-311.
10. Khvatova E.M., Samartzev V.N., Zagoskin P.P., Prudchenko I.A., Mikhaleva I.I. Delta sleep inducing peptide (DSIP): effect on respiration activity in rat brain mitochondria and stress protective potency under experimental hypoxia. Peptides, 2003 (in press).
11. Makletsova M.G., Kharin V.G., Kuraev E.G., Mikhaleva I.I., Prudchenko I.A. Preparation for treatment of burn induced wounds // Patent of Russian Federation N RU 2070054 C1.
12. Makletsova M.G., Rikhireva G.T., Mendzherkitskii A.M., Kolesnikova L.V., Kopylovskii S.A., Mikhaleva I.I. Influence of DSIP upon glutamine synthetase activity in the rat cerebral cortex in normal and immobilized rats. // Neurochemistry (Russian.) - 1995. - V. 12. - p.34-39.
13. Mikhaleva I.I., Prudchenko I.A., Mendzherkitskii A.M., Vilkov G.A. Peptides with antistressor, anticonvulsant and neuroprotective action. // Patent of Russian Federation. - Application number 9701435/04 from 28.01/97.
14. Prudchenko I.A., Efremova E.V., Mikhaleva I.I., Shandra, Godlevsky L.S., Vastyanov R.S. Structure-Activity Studies of Delta Sleep Inducing Peptide (DSIP) Analogues on an Animal Model of Epilepsy. // Peptides. - 1996.
15. Popovich I.G., Vojtenkov B.O., Anisimov V.N., Zabezhinskij M.A., Mikhalev I.I., lvanov V.T.Vlijanie of a peptide delta of sleep on lifetime and development of spontaneous tumours in mice. // Reports of the Academy of sciences. 2003. - vol.388 (5.) - p.701-703.
16. Krichevskaia A.A., Bondarenko T.I., Krupennikova E.I., Mikhaleva I.I. Effect of delta-sleep-peptide on the state of brain membranes during exposure to cold stress. // Fiziol Zh SSSR 1986. - V.72 (6.) - p.843-845.
17. Mikhaleva I., Prudchenko I., and Ivanov V. Delta-sleep Inducing Peptide (DSIP) and its Analogues: Sleep and Extra sleep actions. // Peptides. - 1992, C.H.Schneider, A.N.Eberle (Eds.) Escom, 1993, p.663-664.
18. Mikhaleva I., Prudchenko I., Rikhireva G., Kopylovskii S., Golubev I. // J. Pept. Sci. 2000. Supplement to V.6. - p. 305.
19. Bondarenko T.I., Milyutina N.P., Shustanova T.A., Mikhaleva I.I. The effects of delta sleep-inducing peptide on the intensity of lipid peroxidation and xanthine oxidase activity in rat tissues during cold stress // Neurosci Behav Physiol. 2001. - V.31 (1.) - p.83-86.
20. Popovich I.G. et al. Mechanisms of Aging and Development. 124 (2003) 721-731.
21. Patent of the Russian Federation Nº 2084238, A61 K 38/08 // (A61 K 38/08, 38/05, 31 /195), 1997.
22. US patent application Nº 20010053797 A1, A01 N 037/12; A01 N 037/44 (514/562), 2001.
23. US patent application Nº 20040223963, A61 K 038/46; A61 K 031/405 (424/94.65; 514/414; 514/565), 2004.
24. Patent of the Russian Federation Nº 2099078, A61 K 38/08 // (A61 K 38/08, 31/195), 1997.

## Claims

1. An agent for correction of stress-induced neurotransmitter, neuroendocrinological and metabolic disorders, comprising peptides corresponding to the general formulae listed below and forming the family of delta-sleep peptide (DSIP or Trp-Ala-Gly-Gly-Asp-Ala-Ser-Gly-Glu), DSIP analogues and derivatives containing 4 - 9 or more amino acid residues:
[I] X-Y¹-Y²-Y³-Y⁴-R
[II] X-Y¹-Y²-Y³-Y⁴-Y⁵-R
[III] X-Y¹-Y²-Y³-Y⁴-Y⁵-Y⁶-R
[IV] X-Y¹-Y²-Y³-Y⁴-Y⁵-Y⁶-Y⁷-R
[V] X-Y¹-Y²-Y³-Y⁴-Y⁵-Y⁶-Y⁷-Y⁸-R
[VI] X-Y¹-Y²-Y³-Y⁴-Y⁵-Y⁶-Y⁷-Y⁸-Y⁹-R
where
X = H or acyl residue of formic, acetic, propionic or an other fatty acid, and their aryl derivatives,
Y¹ = Trp or Tyr residue of L- or D-configuration or corresponding N-methyl derivative,
Y² = Ala, N-Me-Ala, Gly, Pro, or hydroxyl-proline residue of L- or D-configuration,
Y³ = Gly or Ala, N-Me-Ala, Pro or hydroxyl-proline residue of L- or D-configuration,
Y⁴ = Gly or Ala, N-Me-Ala, Pro or hydroxyl-proline residue of L- or D-configuration,
Y⁵ = Asp, Glu residue or corresponding N-methyl derivative of L- or D-configuration,
Y⁶ = Ala residue or other protein amino acid or hydroxyl-proline residue of L- or D-configuration or corresponding N-methyl derivative,
Y⁷ = Ser residue or other protein amino acid or hydroxyl-proline residue of L- or D-configuration or corresponding N-methyl derivative,
Y⁸ = Gly residue or other protein amino acid or hydroxyl-proline residue of L- or D-configuration or corresponding N-methyl derivative,
Y⁹ = Glu residue or other protein amino acid or hydroxyl-proline residue of L- or D-configuration or corresponding N-methyl derivative,
R = -OH, -OR₁, -NH-R₁, (where R₁ = H or -alkyl, arylalkyl).

2. The agent according to claim 1, **characterized in that** the delta-sleep peptide family comprises peptides [I-VI], extended at the N- or C-terminus by polypeptide chains composed of 1 - 9 protein amino acid residues, wherein N- and C- terminal residues of the extended peptides may be free or may be blocked by X and/or R groups.

3. The agent according to claim 1 or 2, **characterized in that** the delta-sleep peptide family also comprises multimeres of family peptides and peptides associated with different carriers (for example, polyethylene glycol).

4. The agent according to claim 1 or 2 or 3, **characterized in that** the medical compositions comprise the abovementioned peptide derivatives and one or more other synergistically acting components, such as various protein amino acids, e.g. the inhibitory neurotransmitter glycine, natural direct antioxidants, such as carnosine, homocarnosine or precursors and corresponding acetyl derivatives thereof.

5. The agent according to claim 1 or 2 or 3 or 4, **characterized in that** compositions may also comprise one ore more various other neuroprotective metabolically active components: phospholipids, e.g. lecithin, unsaturated fatty acids, including ω-3-fatty acid derivatives, choline and its derivatives, trimethylglycine, vitamin C, vitamin A and/or precursors thereof, nicotine amide (vitamin PP), vitamin E (alpha-tocopherol), vitamins of the B group (B₁, B₆, B₁₂, B₃), folic acid, alpha-lipoic acid, eicosapentaenoic acid and docosahexaenoic acid.

6. The agent according to claim 1 or 2 or 3 or 4 or 5, **characterized in that** compositions may also comprise various plant bioflavonoides, coenzyme Q10, taurine, terpenes, polyphenols, plant neutriceuticals on the basis of extracts from garlic, onion, green tea, ginseng, grapes, licorice, ginger, *Gotu kola, gingko biloba*, coniferous plants, and also succinic acid, *Riboxinum,* macro- and trace elements (magnesium, potassium, calcium, chrome, selenium ions, etc.) etc.

7. The agent according to claim 1 or 2 or 3 or 4 or 5 or 6, **characterized in that** compositions may comprise other acceptable ingredients, commonly used as fillers for the preparation of drug products, in particular bestatin or other inhibitors of aminopeptidases.

8. The agent according to claim 1 or 2 or 3 or 4 or 5 or 6 or 7, **characterized in that** the weight ratio of the main active components of the composition varies in the following range: peptide component : amino acid component : natural antioxidant = 1 : (0-250) : (0-250).

9. Method of prophylaxis of the development of a pathological stress-syndrome under significant psychoemotional and mental stress by exam or test preparation by way of administering a medicament, **characterized in that** the agent according to claim 1 or 2 or 3 or 4 or 5 or 6 or 7 or 8 is administered intranasally in a dose of at least 0.3 mg two times per day for at least 10 days to adults and in a dose of at least 1 ampoule per day for at least 10 days to children depending on their age.

10. Method of prophylaxis of the development of a pathological stress-syndrome and its complications under significant emotional and physical stress by long-distance passages and long-distance flights, by frequent changes of time zones and of the communication language, **characterized in that** the agent according to claim 1 or 2 or 3 or 4 or 5 or 6 or 7 or 8 is administered intranasally and/or sublingually in a dose of at least 0.3 mg one time per day.

11. Method of prophylaxis of significant psychological and physical stress caused by sport competitions on a professional and/or amateur level and intensive trainings, **characterized in that** the agent according to claim 1 or 2 or 3 or 4 or 5 or 6 or 7 or 8 is administered intranasally and/or sublingually or intravenously in a dose of at least 0.3 mg once per day for at least 5 days with repeated courses.

12. Method of prophylaxis and reduction of manifestations of acute stress condition and symptoms of exhausting stress overstrain and borderline neuropsychic pathology, **characterized in that** the agent according to claim 1 or 2 or 3 or 4 or 5 or 6 or 7 or 8 is administered intranasally and/or sublingually or intravenously in a dose of least 0.3 mg once per day for at least 5 days.

13. Method of prophylaxis and reduction of manifestations of acute stress condition and symptoms of exhausting stress overstrain in extreme conditions of military operations, **characterized in that** the agent according to claim 1 or 2 or 3 or 4 or 5 or 6 or 7 or 8 is administered intranasally and/or sublingually or intravenously at least once per day for at least 5 days.

14. Method of reduction of the withdrawal syndrome in the therapy of opioid addiction, **characterized in that** the agent according to claim 1 or 2 or 3 or 4 or 5 or 6 or 7 or 8 is administered intravenously in a dose of at least 0.3 mg at least 4 times per day for at least 3 days.

15. Method of complex treatment of the opioid abstinence syndrome, **characterized in that** the agent according to claim 1 or 2 or 3 or 4 or 5 or 6 or 7 or 8 is administered intranasally in a dose of least 0.3 mg per day for at least 3 days.

16. Method of complex treatment of the long-term stress disorders with the purpose of correction of the neurotic disorder and of reduction of neurotic manifestations by neurasthenia with anxious-phobic disorders, alcoholism and opioid addiction, **characterized in that** the agent according to claim 1 or 2 or 3 or 4 or 5 or 6 or 7 or 8 is administered intranasally in a course dose of at least 60.0 mg (6 mg per day for at least 10 days) without simultaneous use of other drugs.

17. A method of reduction of ethanol attraction and the withdrawal syndrome of associated forms of alcoholism and opioid narcomania, **characterized in that** the agent according to claim 1 or 2 or 3 or 4 or 5 or 6 or 7 or 8 is administered intranasally in a dose of at least 3 mg/day.

18. Method of prophylaxis of functional disorders of sexual activity and of increasing sexual potency, in particular, in elderly people and under conditions of chronic stress, **characterized by** a long-term administration of the agent according to claim 1 or 2 or 3 or 4 or 5 or 6 or 7 or 8 in a dose of at least 3 mg/day for at least 2 weeks with repeating courses of at least 2 times a year.

19. Method of prophylaxis and treatment of different manifestations of the metabolic syndrome, in particular, in cardiovascular system pathology, **characterized in that** the agent according to claim 1 or 2 or 3 or 4 or 5 or 6 or 7 or 8 is administered intranasally to patients with a severe progressing course of ischemic heart disease against a background of hypertonic disease of grade II-III in a dose of least 3 - 6 mg/day for at least 7 days.

20. Method of prophylaxis of complications associated with coronarographic studies, **characterized in that** the agent according to claim 1 or 2 or 3 or 4 or 5 or 6 or 7 or 8 is administered intranasally in a dose of at least 3 mg/day at least 3 days before a coronary angiography.

21. Method of reducing the dose of long-term applied cardiotropic drugs in elderly patients older than 70 years with an ischemic heart disease, **characterized in that** the agent according to claim 1 or 2 or 3 or 4 or 5 or 6 or 7 or 8 is administered intranasally in a dose of at least 3 mg/day for at least the first 5 days of each month.

22. Method of prophylaxis and treatment of middle aged patients suffering from diabetes mellitus of type II of medium severity by way of course treatment of a medicament, **characterized in that** the agent according to claim 1 or 2 or 3 or 4 or 5 or 6 or 7 or 8 is administered in a dose of at least 3 mg two times per day for the first six days and subsequently in a dose of at least 3 mg once in the morning for at least 8 days until a satisfactory compensation of the diabetes mellitus is achieved, or in case of repeated course administration of the drug until stabilization of the carbohydrate metabolism at an acceptable level for a significant time is achieved.

23. Method of treatment of elderly people with diabetes mellitus of type II by way of administering a medicament, **characterized in that** the agent according to claim 1 or 2 or 3 or 4 or 5 or 6 or 7 or 8 is administered intranasally in a dose of at least 3 mg per day for at least 20 days until a subjective improvement of life quality is achieved.

24. Method of reducing neurocirculatory dystonia and discirculatory encephalopathy of atherosclerotic genesis in case of degradation of the memory and mental working capacity and computer-based stress by way of administering a medicament, **characterized in that** the agent according to claim 1 or 2 or 3 or 4 or 5 or 6 or 7 or 8 is administered in a dose of at least 3 mg per day for at least the first 5 days of each month and subsequently for at least the first 5 days of every quarter.

25. Method of treatment of vegetative vascular dystonia in order to improve sleep quality, to reduce the cephalic syndrome, to decrease the frequency of migraine attacks, to decrease anxiety, irritability and mood fluctuations by way of administering a medicament, **characterized in that** the agent according to claim 1 or 2 or 3 or 4 or 5 or 6 or 7 or 8 is administered in a dose of at least 3 mg per day for at least 5 days, with at least two times repeated analogous course administration.

26. Method of prophylaxis and treatment of patients suffering from migraine,
**characterized in that** the agent according to claim 1 or 2 or 3 or 4 or 5 or 6 or 7 or 8 is administered in a dose of at least 3 mg per day for at least 7 days.

27. Method of treatment of patients with moderate and severe bronchial asthma by way of administering a medicament, **characterized in that** the agent according to claim 1 or 2 or 3 or 4 or 5 or 6 or 7 or 8 is administered in a dose of at least 6 mg per day for five days, and subsequently in a dose of at least 3 mg per day (in the evening) for 20 days until a significant reduction of the need for use of beta-agonists in patients by way of reducing bronchial obstruction is achieved.

28. Method of treatment of acute pancreatitis and of prophylaxis of complications thereof, **characterized in that** the agent according to claim 1 or 2 or 3 or 4 or 5 or 6 or 7 or 8 is administered intranasally or intravenously in a dose of at least 6 mg per day for five days., which reduces significantly the content of non-peptide components of medium weight molecules that play a role in the development of endotoxicosis.

29. Method of treatment of neuroborreliosis by way of administering a medicament, **characterized in that** the agent according to claim 1 or 2 or 3 or 4 or 5 or 6 or 7 or 8 is administered intranasally in a course dose of at least 60 mg (20 ampoules) until disappearance of complaints regarding irritability, sweating, headache and sleep disturbance.

30. Method of treatment of multiple sclerosis by way of incorporation of a drug into a complex therapy, **characterized in that** the agent according to claim 1 or 2 or 3 or 4 or 5 or 6 or 7 or 8 is administered for at least 10 days until an achievement of positive emotional state dynamics or of a reduction of vegetative dystonia and neurocirculatory dystonia of the hypotonic type.

31. Method of administrating a medicament in treatment of autoimmune thyroiditis, **characterized in that** the agent according to claim 1 or 2 or 3 or 4 or 5 or 6 or 7 or 8 is administered intranasally in a dose of 3 mg for at least 10 - 14 days until a decrease of the thyroid tissue antibody concentration and a trend towards normalization of thyroid hormone levels is achieved.

32. Method of treatment of patients with an infantile cerebral paralysis, including such spastic forms as spastic diplegia, hemiparetic form, atonic-astatic form and also consequences of cranio-cerebral trauma by a medicament, **characterized in that** the agent according to claim 1 or 2 or 3 or 4 or 5 or 6 or 7 or 8 is administered intranasally in a dose of at least 3 mg for at least five days with a subsequent repetition of the courses at least quarterly.

33. Method for the rehabilitation of children with a minimal brain dysfunction and with functional and organic abnormalities of the central nervous system function, **characterized in that** the agent according to claim 1 or 2 or 3 or 4 or 5 or 6 or 7 or 8 is administered intranasally or sublingually in a dose of at least 3 mg for at least 10 days.

34. Method for the rehabilitation of children with a minimal brain dysfunction and with functional abnormalities of the central nervous system function,
**characterized in that** the agent according to claim 1 or 2 or 3 or 4 or 5 or 6 or 7 or 8 is added to products for children's nutrition in a dose of at least 3 mg per ingestion for at least 10 days.

35. Method of treatment of clinical manifestations of a preinatal pathology (abundant regurgitation within one hour and more after feeding, head-tilt in sleep, increased pulsation of the anterior fontanel, muscular hypotonicity or hypertonicity, superficial sleep, day and night confusion) by administering a drug in case of breast feeding, **characterized in that** the agent according to claim 1 or 2 or 3 or 4 or 5 or 6 or 7 or 8 is administered intranasally to breast fed children in a dose of at least 1 ampoule per day for at least 5 days and to bottle-fed children in a dose of at least 11/2 ampoule per day intranasally until achieving a significant positive dynamics, or in case of repeated treatment courses until improvement of indices of physical, psychic and emotional development of the children.

36. Method of treatment of patients suffering from symptomatic and idiopathic epilepsy, **characterized in that** the agent according to claim 1 or 2 or 3 or 4 or 5 or 6 or 7 or 8 is administered in a dose of at least 6 mg per day for at least 20 days.

37. Method of rapid reduction of the manifestations of the withdrawal syndrome in pregnant women actively taking heroin, **characterized in that** the agent according to claim 1 or 2 or 3 or 4 or 5 or 6 or 7 or 8 is administered intravenously or intranasally in a dose of at least 6 mg per day during the drug withdrawal and at least for 20 days during pregnancy and/or prepartum, which results **in that** newborn children did either not develop an abstinence syndrome or developed an abstinence syndrome in a form that did not required any treatment.

38. Method of the symptomatic and pathogenetic treatment of oncologic patients by administering a medicament, **characterized in that** the agent according to claim 1 or 2 or 3 or 4 or 5 or 6 or 7 or 8 is administered intranasally in a dose of at least 0.3 mg per day for 7 days until reduction of the necessary dose of narcotic and non-narcotic analgetics.

39. Method of correction of the polychemotherapy-induced pancytopenia, **characterized in that** the agent according to claim 1 or 2 or 3 or 4 or 5 or 6 or 7 or 8 is administered in pediatric and adult oncologic practice and in early forms of pancytopenia, starting with a minimal dose of 3 mg per day for at least 5 days.

40. Method of complex treatment of burn diseases in pediatric practice of children with a damage area of more than 47% of the body surface and a marked septic toxemia, **characterized in that** the agent according to claim 1 or 2 or 3 or 4 or 5 or 6 or 7 or 8 is administered intranasally in a daily dose of at least 3 mg.

41. Method of treatment of small burn-induced damage (at least 1 % of the body surface) in children and adults, **characterized in that** The agent according to claim 1 or 2 or 3 or 4 or 5 or 6 or 7 or 8 is administered intranasally in a daily dose of at least 3 mg.

42. Method of complex treatment of a life-threatening severe cranio-cerebral trauma by administering a medicament, **characterized in that** the agent according to claim 1 or 2 or 3 or 4 or 5 or 6 or 7 or 8 is administered intranasally in a high dose of up to 15 mg per day and in the case of long-term control of patients after a severe cranio-cerebral trauma with a surgical removal of the crush injury locus of the brain, the agent according to claim 1 or 2 or 3 or 4 or 5 or 6 or 7 or 8 is administered in a dose of at least 3 mg twice per day for at least 5 days in the first month until disappearance of pathological phenomena or normalization of sleep.

43. Method of complex treatment of patients with a diffuse axonal injury in a state of deep coma, **characterized in that** the agent according to claim 1 or 2 or 3 or 4 or 5 or 6 or 7 or 8 is administered intranasally or intravenously in a dose of at least 3 mg per day for at least one week.

44. Method of complex treatment of central and peripheral nervous system pathologies (meningitis, encephalitis of any genesis, paresis, and insults), **characterized in that** the agent according to claim 1 or 2 or 3 or 4 or 5 or 6 or 7 or 8 is administered intranasally or intravenously in a dose of at least 3 ampoules of the drug per day in emergency situations (shock of any genesis) until recovery from the critical condition.

45. Method of elevating the wound healing and of prophylaxis and reduction of the risk of post-surgical complications , including the treatment of gerontological patients, by administering a medicament, **characterized in that** the agent according to claim 1 or 2 or 3 or 4 or 5 or 6 or 7 or 8 is administered intranasally in a dose of at least 3 mg per day for at least 5 days before and/or after a surgical intervention.

46. Method of prophylaxis and reduction of the risk of post-surgical complications in case of planned operations, **characterized in that** the agent according to claim 1 or 2 or 3 or 4 or 5 or 6 or 7 or 8 is administered intranasally in a dose of at least 3 mg per day for at least 5 days before a surgical intervention.

47. Method of prophylaxis and reduction of the risk of post-surgical complications in case of planned operations with an artificial blood circulation, **characterized in that** the agent according to claim 1 or 2 or 3 or 4 or 5 or 6 or 7 or 8 is administered intranasally in a dose of at least 3 mg per day for at least 5 days before the surgery, and intranasally or intravenously in a dose of at least 3 mg per day during the entire pre- and postoperative period.

48. Method of complex treatment of patients with pyoinflammatory diseases of the maxillofacial region, including those with a severe background pathology, **characterized in that** the agent according to claim 1 or 2 or 3 or 4 or 5 or 6 or 7 or 8 is administered intranasally in a dose of at least 3 mg per day during the entire acute period of purulent inflammation.

49. Method of improvement of the skin turgor, of microcirculation, of the ability of the skin to conserve moisture and of resistance to common infections, **characterized in that** the agent according to claim 1 or 2 or 3 or 4 or 5 or 6 or 7 or 8 is administered intranasally in a dose of at least 3 mg per day for 5 - 7 days during a long-term period (half a year and more).

50. Method of significant improvement of the skin turgor, of microcirculation, of the ability of the skin to conserve moisture, and of resistance to common infections, **characterized in that** the agent according to claim 1 or 2 or 3 or 4 or 5 or 6 or 7 or 8 is incorporated into the composition of creams, including creams for the skin treatment around the eyes, of tonics, lotions and milk for skin treatment of the face and the body in a dose of at least 3 mg per volume of the cosmetic for one skin treatment application, wherein the course treatment is performed for at least for one week, and wherein the preparation is incorporated into the composition of creams, tonics, lotions and milk in a dose of at least 6 mg per volume of the cosmetic for one skin treatment application in order improve to the status of the body and facial skin and the periorbital region, including skin treatment around the eyes.

51. Method of treatment of animals with infectious, parasitogenic, noncontagious internal and surgical diseases by administering a medicament, **characterized in that** the agent according to claim 1 or 2 or 3 or 4 or 5 or 6 or 7 or 8 is administered intranasally or subcutaneously in a dose of least 1 mg per day for at least 2 days.

52. Method of increasing the therapeutic effect of the agent according to claim 1 or 2 or 3 or 4 or 5 or 6 or 7 or 8, **characterized in that** the synergistically acting amino acid glycine and/or its derivative trimethylglycine are incorporated into the composition of the agent in an effective combination, which may be applied for a short treatment course of at least 1 day.

53. Method of improvement of the appetite, of growth acceleration and body weight gain in anorexia caused by nervous and physical exhaustion, or after previous diseases and surgeries, or in chronic ischemic heart disease, or for normalization of hyperthyroidism metabolism ,
**characterized in that** the agent according to claim 1 or 2 or 3 or 4 or 5 or 6 or 7 or 8 is administered intranasally or subcutaneously or intramuscularly or intravenously in a dose of at least 3 mg per day at least 1 - 2 times per day for at least 5 days in combination with biogenic substances such as carnitine, carnosine, homocarnosine or precursors thereof.

54. Method for the improvement of carbohydrate metabolism especially in case of diabetes mellitus type II in middle-aged and elderly patients and also in case of liver diseases, peptic ulcers of the stomach and the duodenum, **characterized in that** the agent according to claim 1 or 2 or 3 or 4 or 5 or 6 or 7 or 8 should be administered intranasally or subcutaneously or intramuscularly or intravenously in a dose of at least 3 mg per day at least 1 - 2 times per day for at least 10 days in combination with nicotinamide, or vitamin PP being a hydrogen carrier and effecting oxidation-reduction-processes.

55. Method of treatment of slow-healing ulcers and wounds and of varicose veins of the inferior extremities, **characterized in that** the agent according to claim 1 or 2 or 3 or 4 or 5 or 6 or 7 or 8 is administered intranasally or intramuscularly in a dose of at least 3 mg per day at least 1 - 2 times per day for at least 5 days in combination with nicotinamide or vitamin PP, or the agent according to claim 1 or 2 or 3 or 4 or 5 or 6 or 7 or 8 is incorporated into preparative combinations for the local treatment of wound surfaces in a dose of at least 6 mg per volume of the agent for ligation.

56. Method of treatment of neuritis, radiculitis, neuralgia and peripheral paralysis, **characterized in that** the agent according to claim 1 or 2 or 3 or 4 or 5 or 6 or 7 or 8 is administered intranasally or intramuscularly in a dose of at least 3 mg per day at least 1 - 2 times per day for at least 5 days in combination with vitamin B₁ (Thiaminum) that supports a faster resolution of the acute symptoms and a reduction of the hospitalization period and invalidism.

57. Agent for the enhancement of the metabolism of tryptophane, methionine, cystein, glutamic and other amino acids, **characterized in that** the agent according to claim 1 or 2 or 3 or 4 or 5 or 6 or 7 or 8 is administered intranasally, intramuscularly or intravenously in a dose of at least 3 mg per day at least 1 - 2 times per day for at least 5 days in combination with vitamin B₆ (pyridoxine) and derivatives thereof that supports resolution of pregnancy toxemia and improvement of the condition of the fetus in water rich pregnancy.

58. Method of increasing the efficacy of recombinant growth factors in leukopenic conditions , **characterized in that** the agent according to claim 1 or 2 or 3 or 4 or 5 or 6 or 7 or 8 is administered intranasally, intramuscularly or intravenously in a dose of at least 3 mg per day at least 1 - 2 times per day for at least 5 days in combination with vitamin B₆ (pyridoxine) and derivatives thereof that supports reduction of the course of administration of recombinant drugs while keeping their efficacy.

59. Method of increasing the efficacy of a combined treatment of Ménière's disease and depressions of elderly people, **characterized in that** the agent according to claim 1 or 2 or 3 or 4 or 5 or 6 or 7 or 8 is administered intranasally, intramuscularly or intravenously in a dose of at least 3 mg per day at least 1 - 2 times per day for at least 10 days in combination with vitamin B₆ (pyridoxine) and derivatives thereof.

60. Method of prophylaxis treatment of sea and air sickness, **characterized in that** the agent according to claim 1 or 2 or 3 or 4 or 5 or 6 or 7 or 8 is administered intranasally or intramuscularly in a dose of at least 3 mg per day at least 1 - 2 times per day for at least 1 - 2 days in combination with vitamin B₆ (pyridoxine) and derivatives thereof.

61. Method of reduction of painful syndrome in treatment of diseases of the central nervous system, of traumatic diseases of peripheral nerves, and of the reduction of the frequency of migraine attacks, **characterized in that** the agent according to claim 1 or 2 or 3 or 4 or 5 or 6 or 7 or 8 is administered intranasally, intramuscularly or intravenously in combination with vitamin B₁₂ (cyancobalamin) or derivatives thereof in a dose of at least 3 mg per day at least 1 - 2 times per day until reduction of the painful syndrome or for at least 5 days per month.

62. Method of reduction or prevention of fatty liver infiltration in Botkin's disease, hepatitis, liver cirrhosis (mainly in early stages), hypothyroidism, cysteinuria, and chronic alcoholism, **characterized in that** the agent according to claim 1 or 2 or 3 or 4 or 5 or 6 or 7 or 8 is administered intranasally, intramuscularly or intravenously in combination with phosphatide lecithine and its constituent choline in a dose of at least 3 mg per day at least 1 - 2 times per day for at least 10 days with repeated courses of at least two times a year.

63. Method of prophylaxis and treatment of coronary atherosclerosis, liver diseases (light or middle-severe Botkin's disease - in absence of an intensive or increasing jaundice; chronic hepatitis, cirrhosis), diabetic polyneuritis, and intoxications, **characterized in that** the agent according to claim 1 or 2 or 3 or 4 or 5 or 6 or 7 or 8 is administered intranasally, intramuscularly or intravenously in combination with lipoic acid in a dose of at least 3 mg per day at least 1 - 2 times per day for at least 10 days with repeated courses at least 3 times a year.

64. Method of prophylaxis and treatment of scurvy, hemorrhagic diathesis, of nasal, parenteral, pulmonary and other hemorrhages, of an overdosage of anticoagulants, of contagious diseases, of slow-healing wounds and fractures of bones, **characterized in that** the agent according to claim 1 or 2 or 3 or 4 or 5 or 6 or 7 or 8 is administered intranasally, intramuscularly or intravenously in combination with ascorbic acid in a dose at of least 3 mg per day at least 1 - 2 times per day for at least 5 days.

65. Method of improvement of the tolerance to serious physical stress and strain, including in pregnancy, **characterized in that** the agent according to claim 1 or 2 or 3 or 4 or 5 or 6 or 7 or 8 is administered intranasally, intramuscularly or intravenously in combination with ascorbic acid in a dose of at least 3 mg per day at least 1 - 2 times per day for at least 5 days.

66. Method of reducing the severity of clinical manifestations and increasing duration and stability of neurologic remission in nervimuscular diseases (e.g. muscle dystrophy, amyotrophic lateral sclerosis, etc.), **characterized in that** the agent according to claim 1 or 2 or 3 or 4 or 5 or 6 or 7 or 8 is administered intranasally, intramuscularly or intravenously in combination with vitamin E (alpha-Tocopherol) in a dose of at least 3 mg per day at least 1 - 2 times per day for at least 10 days with repeated courses at least 3 times per year.

67. A method for recovery of spermatogenesis, potency and normal erectile function, **characterized in that** the agent according to claim 1 or 2 or 3 or 4 or 5 or 6 or 7 or 8 is administered intranasally or intramuscularly in combination with the vitamin E (alpha-Tocopherol) in a dose of at least 6 mg per day at least 1 - 2 times per day for at least 5 days with repeated courses at least 3 times per year.

68. Method of reduction of the volume of therapeutic interventions in skin damages and skin diseases (chillblain, wounds, fish-skin disease, follicular dyskeratosis, senile keratosis), **characterized in that** the agent according to claim 1 or 2 or 3 or 4 or 5 or 6 or 7 or 8 is administered intranasally or intramuscularly in combination with vitamin A (Retinol) and/or precursors thereof in a dose of at least 3 mg per day at least 1 - 2 times per day for at least 10 days with repeated courses at least 3 times per year.

69. Method of reduction of the volume of therapeutic interventions in ischemic heart disease (chronic coronary failure and myocardial infarction) and in myocardial dystrophy, **characterized in that** the agent according to claim 1 or 2 or 3 or 4 or 5 or 6 or 7 or 8 is administered intranasally or intramuscularly in combination with Riboxinum (a derivative of the nucleoside purine) in a long-term dose of at least 3 mg per day at least 1 - 2 times per day for at least 10 days with repeated courses at least 3 times per year.

70. Method of significant improvement of metabolism of tissues exposed to oxidative stress, **characterized in that** the agent according to claim 1 or 2 or 3 or 4 or 5 or 6 or 7 or 8 is administered intranasally or intramuscularly in combination with vegetable bioflavonoides in long-term a dose of at least 3 mg per day at least 1 - 2 times per day for at least 1 - 3 days with intervals between courses of up to 4 weeks and with repeated courses at least 3 times per year), which stimulates and accelerates tissue repair processes.

71. Method of improvement of liver metabolism in acute and/or chronic intoxication (including alcoholic intoxication) and of functional disorders of the liver caused by hepatitis, **characterized in that** the agent according to claim 1 or 2 or 3 or 4 or 5 or 6 or 7 or 8 is administered intranasally or intramuscularly in combination with succinic acid and derivatives thereof, and also with macroelements and trace elements (e.g. magnesium, potassium, calcium, chrome, selenium ions, etc.) in a dose of at least 3 mg for 3 days with intervals between courses of up to 4 weeks and with a repeated of courses at least once in three months.

72. Method of improvement of tolerance to intense physical stress and of resistance to air pollution by toxic waste products (including conditions of living in a megacity), **characterized in that** the agent according to claim 1 or 2 or 3 or 4 or 5 or 6 or 7 or 8 is administered intranasally or intramuscularly in combination with succinic acid and derivatives thereof, and also with macroelements and trace elements (e.g. magnesium, potassium, calcium, chrome, selenium ions, etc.) in a dose of at least 3 mg for at least 3 days with intervals between courses of about 4 weeks and repeated courses of at least once in three months.

73. Method of conservation and maintenance of mnestic functions in conditions of sustained attention, **characterized in that** the agent according to claim 1 or 2 or 3 or 4 or 5 or 6 or 7 or 8 is administered intranasally or intramuscularly in combination with succinic acid and derivatives thereof, and also with macroelements and trace elements (e.g. magnesium, potassium, calcium, chrome, selenium ions, etc.) in a dose of at least 3 mg for 1 - 5 days with intervals between courses of about 4 weeks and repeated courses at least once in three months).
